# EUROPEAN PATENT APPLICATION

(11) **EP 0 725 068 A1**
(43) Date of publication of application: **07.08.1996**
(21) Application number: 94930346.5
(22) Date of filing: 20.10.1994
(51) Int. Cl.: C07D 471/06, A61K 31/475

(54) **INDOLE DERIVATIVE**

(30) Priority: 20.10.1993 JP 261997/93
(71) Applicant: TOKYO TANABE COMPANY LIMITED, Chuo-ku Tokyo 103 (JP)
(72) Inventor: TSUCHIYA, Shinji Tokyo Tanabe Company Limited, Akabane-kita 2-chome Kita-ku Tokyo 115 (JP); YASUDA, Noboyuki Tokyo Tanabe Company Limited, Akabane-kita 2-chome Kita-ku Tokyo 115 (JP); FUKUZAKI, Atsushi Tokyo Tanabe Company Limited, Akabane-kita 2-chome Kita-ku Tokyo 115 (JP)
(74) Representative: Calamita, Roberto
(86) International application number: JP9401769
(87) International publication number: WO9511245

(57) **Abstract**

Indole derivatives represented by the general formula:
31) 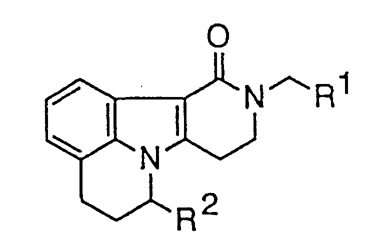 wherein R¹ represents:
32) 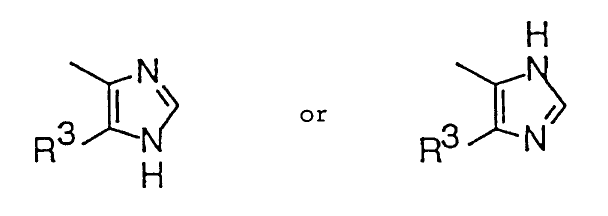 R² represents a phenyl group which may be substituted, and R³ represents a hydrogen atom or a methyl or ethyl group, either as single isomers or as isomer mixtures, as well as physiologically acceptable salts thereof, and their solvates. The indole derivatives of the invention exhibit more powerful and selective antagonism against intestinal 5-HT₃ receptors than known 5-HT₃ receptor antagonists and also have excellent duration of action, making them useful as prophylactic or therapeutic agents against digestive tract function disorders such as irritable bowel syndrome, diarrhea, and the like.

## Description

### Field of the Invention

The present invention relates to a indole derivative which is useful as drug, and specifically as 5-HT₃ receptor antagonist.

### Background of the Art

The 5-HT₃ receptor which is one of the 5-hydroxytryptamine (5-HT, generic name: serotonin) receptors is widely distributed throughout the sensory nervous system, the autonomic nervous system and the central nervous system, and the action of 5-HT on the receptors is said to cause gastrointestinal motor disturbances, irritant mood, vomiting, algesia, bradycardia, and to have nervous activity affecting feelings, appetite, memory, etc. Consequently, drug agents with 5-HT₃ receptor antagonism are reported to be effective for the treatment and prevention of the irritant mood, vomiting, accompanying cancer chemotherapy, migraine, arrhythmia and nervous disorders such as schizophrenia, mania, etc., as well as diarrhea, irritable bowel syndrome, increased urinary frequency and dysuria.

Among such drug agents with 5-HT₃ receptor antagonism there are used 4-amino-5-chloro-N-[2-(diethylamino)ethyl]-2-methoxybenzamide (generic name: "Metoclopramide"), 1,2,3,4-tetrahydro-9-methyl-3-[(2-methyl-1H-imidazol-1-yl)methyl]-4H-carbazol-4-one hydrochloride dihydrate (GR38032F, generic name: "Ondansetron hydrochloride"), endo-8-methyl-8-azabicyclo[3,2,1]octa-3-ylindol-3-ylcarboxylate (ICS 205-930, generic name: "Tropisetron"), N-(endo-9-methyl-9-azabicyclo[3,3,1]non-3-yl)-1-methylindazol-3-carboxamide hydrochloride (generic name: "Granisetron"), etc., which are used to treat vomiting elicited by antitumor agents such as cisplatin and the like, while other compounds such as (R)-(-)-5-[(1-methylindol-3-yl)carbonyl]-4,5,6,7-tetrahydrobenzimidazole hydrochloride (YM-060) and 2,3,4,5-tetrahydro-5-methyl-2-[(5-methyl-1H-imidazol-4-yl)methyl]-1H-pyrido[4,3-b]indol-1-one hydrochloride (GR-68755C, generic name: "Allosetron hydrochloride") are currently receiving much attention.

However, a number of subtypes of the 5-HT₃ receptor are reported to exist. As a result of research focusing on a 5-HT₃ receptor in the intestine, it is believed that this intestinal 5-HT₃ receptor plays a role in various forms of diarrhea, for example stress-related diarrhea and secretory diarrhea caused by cholera and other bacteria, and thus compounds with strong antagonism for the intestinal 5-HT₃ receptor are expected to work as antidiarrheal drugs. It has in fact been reported that the above-mentioned 5-HT₃ receptor antagonists Ondansetron, Granisetron, YM-060, etc. suppress stress-related diarrhea in animal models, with the effect of YM-060 being the strongest (The Journal of Pharmacology and Experimental Therapeutics, Vol.261, No.1, 297 (1992)). However, despite the abundant research on 5-HT₃ receptor antagonists as antiemetics, there has been a little research on their use as antidiarrheal drugs, and the characteristics of colonic 5-HT₃ receptors has not yet been adequately studied. As mentioned above, 5-HT₃ receptors are distributed throughout the body and mediate a large number of effects, and therefore if 5-HT₃ receptor antagonists used as antidiarrheal agents have strong 5-HT₃ receptor antagonism at sites other than the colon, they can block normal function there and may produce aberrations in mental activity, cardiac function, gastrointestinal and other internal organ functions, algesia, and the like.

On the other hand, compounds which are structurally similar to the compounds of the present application are described in Japanese laid-open patent publication No. Hei-2-180885 and No. 2-258785, and in particular the indolo(3,2-C)lactam compounds disclosed in Japanese laid-open patent publication No. Hei-2-258785 also encompass the compounds of the present application. However, these publications do not describe the specific structures, names, physical data and effects of the compounds of the present application, and as will be explained later, they all exhibit low antagonism for 5-HT₃ receptors in the colon.

In view of this, the present inventors have sought to develop a 5-HT₃ receptor antagonist which has a strong antidiarrheal effect, which is safe with few side effects, and which may easily be used as a drug, and as a result of diligent research particularly on compounds exhibiting high antagonism for colonic 5-HT₃ receptors, we have completed the present invention on the discovery of indole derivatives with powerful antagonism for colonic 5-HT₃ receptors and high colon selectivity, as well as a powerful antidiarrheal effect, as compared to known compounds with similar structures and other typical 5-HT₃ antagonists.

### Disclosure of the Invention

The present invention relates to a indole derivative and its individual isomers and isomeric mixture (hereunder collectively referred to as "compound (a)") represented by the following general formula:
1) 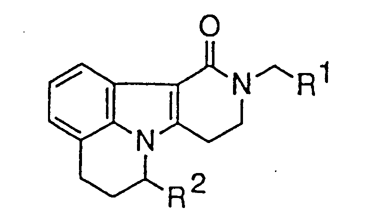 wherein R¹ represents the group
2) 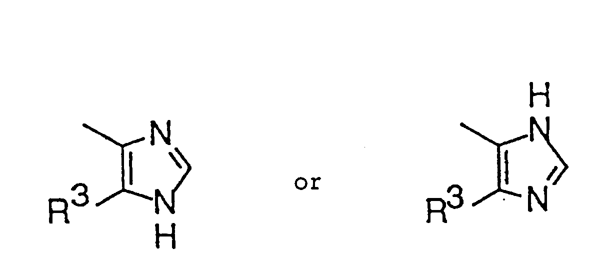 R² represents a phenyl group which may be substituted, and R³ represents a hydrogen atom or a methyl or ethyl group, and to its physiologically acceptable salt and its solvate.

In compound (a), a 5-(R³)-1H-imidazol-4-yl group and 4-(R³)-1H-imidazol-5-yl group represented by R¹ are mutual tautomers, and are essentially synonymous. In the present specification, no particular distinction is made between the two. That is, for convenience the expression "5-(R³)-1H-imidazol-4-yl group" alone may be used, in which case it is intended to include "4-(R³)-1H-imidazol-5-yl group".

As physiologically acceptable salts of compound (a) there may be mentioned salts of inorganic acids such as hydrochloric acid, hydrobromic acid, hydroiodic acid, sulfuric acid, phosphoric acid and nitric acid and salts of organic acids such as acetic acid, carbonic acid, tartaric acid, fumaric acid, maleic acid, oxalic acid, citric acid, malic acid, benzoic acid, methanesulfonic acid, ethanesulfonic acid and benzenesulfonic acid.

When R² is a substituted phenyl group, suitable substituents on the phenyl group may be lower alkyl groups such as methyl, ethyl, propyl and isopropyl, hydroxyl group, lower alkoxy groups such as methoxy, ethoxy, propoxy and isopropoxy, halogens such as fluorine, chlorine and bromine, amino group, lower alkylamino groups, alkylcarbamoyl groups, carbamoyl group, sulfamoyl groups, lower alkoxycarbonyl groups, nitro group, and acylamino groups such as acetylamino and propionylamino.

Compound (a) in which R² is a phenyl group may be produced by reacting the following compound (hereunder referred to as "compound (b)"):
3) 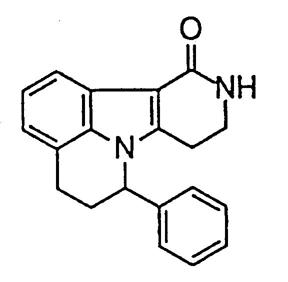 with a compound represented by the following general formula (hereunder referred to as "compound (c)"):
4)

   Cl-CH₂-R⁴

   wherein R⁴ represents a 5-(R³)-1H-imidazol-4-yl group or 4-(R³)-1H-imidazol-5-yl group which may have a protecting group and R³ is the same as defined previously, in the presence of sodium hydride, lithium diisopropylamide or the like, and deprotecting a desired substituent if necessary.

Preferred protecting groups for R⁴ include arylmethyl groups such as trityl and benzyl, acyl groups such as carbobenzyloxy and ethoxycarbonyl, and sulfonyl groups such as toluenesulfonyl and p-methoxytoluenesulfonyl.

Compound (b) may be produced by the method represented by the following reaction process A:
5)

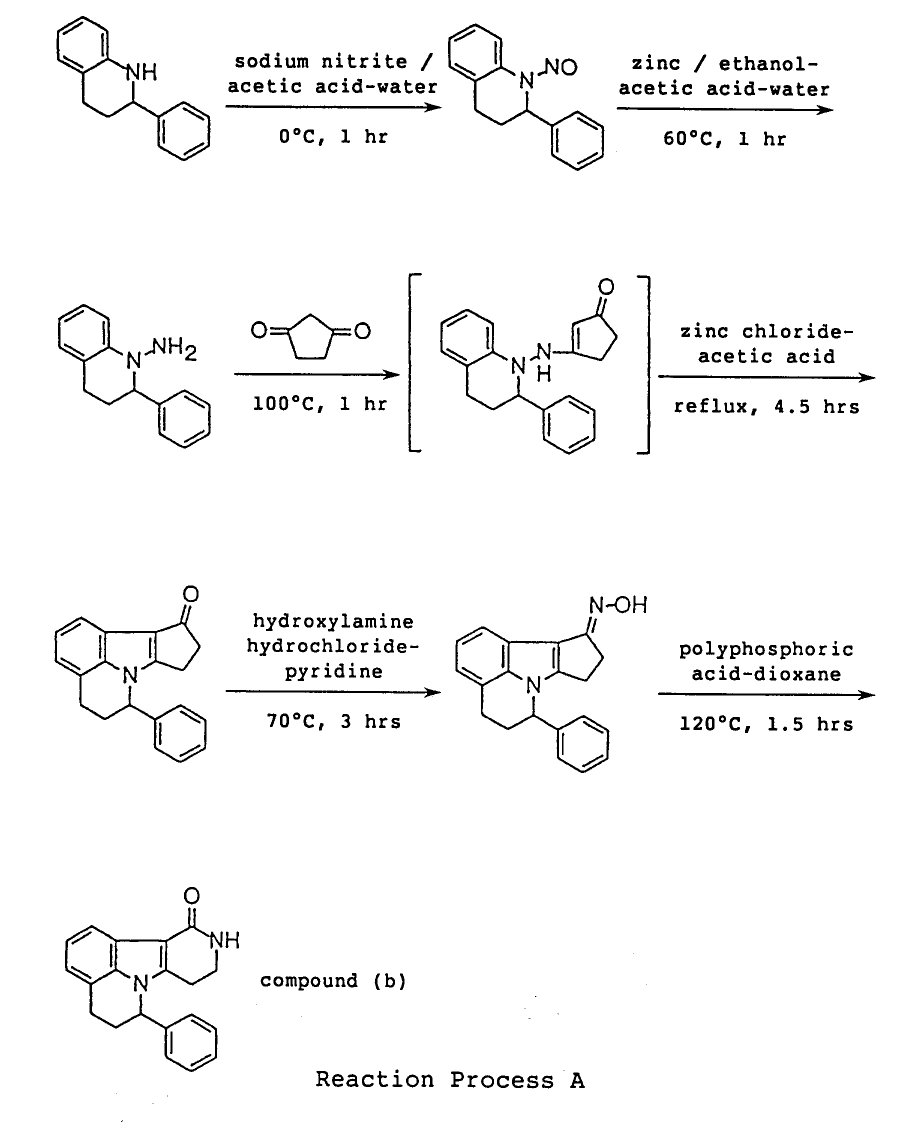

Compound (c) is a publicly known compound and may be produced by, for example, the method described in Japanese laid-open patent publication No. Sho-63-211279.

When R² in compound (a) is a substituted phenyl group, it is prepared as when R² is a phenyl group, but using a substituted starting material, and if necessary converting the substituent by a common organic reaction, or protecting or deprotecting it.

### Brief Description of the Drawings

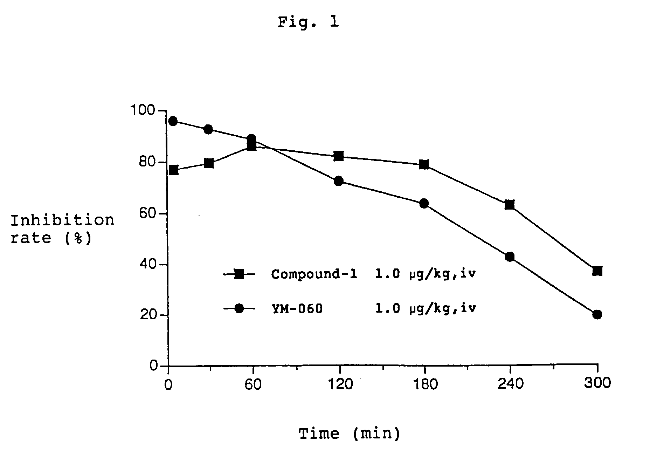

Fig. 1 is a graph showing the results of measuring the duration of action of 5-HT₃ receptor antagonism (Inhibition of BJ reflex) of a compound of the invention and a comparison compound in Test 4.

### Best Mode for Carrying Out the Invention

### [Reference Example 1]

### 5,6,8,9,10,11-hexahydro-6-phenyl-11-oxo-4H-pyrido[3',4':4,5]pyrrolo[3,2,1-ij] quinoline:

A 16.7 g (79.9 mmol) portion of 1,2,3,4-tetrahydroquinoline was dissolved in 150 ml of acetic acid, and 40 ml of an aqueous solution containing 6.08 g (88 mmol) sodium nitrite was added dropwise under ice cooling. After the dropwise addition, the mixture was stirred for one hour at room temperature. The reaction mixture was diluted with water and extracted with ether. After ether extract solution was washed with an aqueous sodium hydroxide solution, it was dried over anhydrous magnesium sulfate and the solvent was distilled off to obtain 17.9 g (75.2 mmol) of 1-nitroso-2-phenyl-1,2,3,4-tetrahydroquinoline as a brown oil (94% yield).
¹H-NMR (δppm, CDCl₃, 270MHz):
2.1∼2.3 (2H, m), 2.6∼2.8 (2H, m), 6.04 (1H, t), 7.0∼7.1 (2H, m), 7.2∼7.4 (6H, m), 8.25 (1H, d)

Fifty grams of powdered zinc was suspended in 75 ml ethanol, 5.0 ml water and 2.5 ml acetic acid and heated to 60°C, and to this suspension a solution of 6.30 g (26.4 mmol) of the above-mentioned 1-nitroso-2-phenyl-1,2,3,4-tetrahydroquinoline in 25 ml ethanol, 2.5 ml water and 20 ml acetic acid was added dropwise. The mixed suspension was heated at 60°C for 30 minutes and then cooled to room temperature, and the insoluble precipitate was filtered out. After making the filtrate basic with an aqueous sodium hydroxide solution, the solution was extracted with ether. The extract solution was dried over anhydrous magnesium sulfate and the solvent was distilled off to obtain a residue. The residue was subjected to silica gel column chromatography, eluted with a mixed solvent of hexane:ethyl acetate=5:1 and then concentrated to obtain 4.10 g (18.3 mmol) of 1-amino-2-phenyl-1,2,3,4-tetrahydroquinoline as a yellow oil (69% yield).
¹H-NMR (δppm, CDCl₃, 270MHz):
2.0∼2.3 (2H, m), 2.6∼2.9 (2H, m), 3.44 (2H, brs), 4.34 (1H, dd), 6.71 (1H, t), 6.98 (1H, d), 7.16 (1H, t), 7.2∼7.4 (6H, m)

A 6.08 g (27.1 mmol) portion of the above-mentioned 1-amino-2-phenyl-1,2,3,4-tetrahydroquinoline was mixed with 3.20 g (33.0 mmol) of 1,3-cyclopentanedione, and the mixture was heated at 100°C for one hour. The reaction mixture was then cooled to room temperature and subjected to silica gel column chromatography, and eluted with a mixed solvent of chloroform:ethanol = 50:1. The elute was concentrated under reduced pressure to give 9.20 g of a brown glassy substance. The glassy substance was dissolved in 250 ml acetic acid, 100 g zinc chloride was added, and the mixture was heated under reflux for 4.5 hours. The reaction solution was poured into ice water and the precipitating crystals were collected by filtration and washed with water. The resultant crystals were subjected to silica gel column chromatography and eluted with chloroform, and the solvent was concentrated distilled off to obtain 3.09 g (10.8 mmol) of 5,6,9,10-tetrahydro-6-phenyl-10-oxo-4H,8H-cyclopenta[4,5]pyrrolo[3,2,1-ij]quinoline as colorless crystals (40% yield).
¹H-NMR (δppm, CDCl₃, 270MHz):
2.3∼2.6 (3H, m), 2.7∼3.0 (5H, m), 5.41 (1H, t), 7.0∼7.1 (3H, m), 7.22 (1H, t), 7.3∼7.4 (3H, m), 7.79 (1H, d)

A 4.00 g (13.9 mmol) portion of the above-mentioned 5,6,9,10-tetrahydro-6-phenyl-10-oxo-4H,8H-cyclopenta[4,5]pyrrolo[3,2,1-ij]quinoline and 3.90 g (55.6 mmol) of hydroxylamine hydrochloride were suspended in 80 ml of pyridine and heated to 70°C with stirring for 3 hours. After cooling the reaction solution to room temperature the pyridine was distilled off under reduced pressure to obtain a residue. To this residue there was added 200 ml of a saturated aqueous sodium hydrogen carbonate solution, and the mixture was extracted with chloroform. Ethanol was added to the residue obtained by concentration of the extract solution under reduced pressure, and the precipitated crystals were collected by filtration. The collected crystals were washed with ethanol and dried to obtain 3.60 g (11.9 mmol) of 5,6,9,10-tetrahydro-6-phenyl-10-hydroxyimino-4H,8H-cyclopenta[4,5]pyrrolo[3,2,1-ij]quinoline as colorless crystals (86% yield).
¹H-NMR (δppm, DMSO-d₆, 270MHz):
2.1∼2.3 (1H, m), 2.3∼2.4 (2H, m), 2.6∼2.8 (2H, m), 2.8∼3.1 (3H, m), 5.57 (0.5H, t), 5.61 (0.5H, t), 6.9∼7.1 (4H, m), 7.3∼7.4 (3H, m), 7.74 (1H, d), 9.94 (0.5H, s), 9.99 (0.5H, s)

A mixture of 120 g of polyphosphoric acid and 60 ml of 1.4-dioxane was heated to 120°C, and to the heated mixture a 3.60 g (11.9 mmol) portion of the above-mentioned 5,6,9,10-tetrahydro-6-phenyl-10-hydroxyimino-4H,8H-cyclopenta[4,5]pyrrolo[3,2,1-ij]quinoline was added in crystal form. After the addition, the heating was continued for one hour and 15 minutes more. After cooling to room temperature, ice water was added and the precipitated crystals were collected by filtration. After washing with water and a saturated aqueous sodium hydrogen carbonate solution, the collected residue was subjected to silica gel column chromatography, eluted with a mixed solvent of chloroform:methanol = 50:1 and concentrated to obtain a residue. Ether was added to the residue and precipitated crystals were collected to give 2.55 g (8.40 mmol) of the 5,6,8,9,10,11-hexahydro-6-phenyl-11-oxo-4H-pyrido[3',4':4,5]pyrrolo[3,2,1-ij] quinoline listed in the above heading as colorless crystals (71% yield).
¹H-NMR (δppm, CDCl₃, 270MHz):
2.4∼3.0 (6H, m), 3.4∼3.7 (2H, m), 5.27 (1H, brs), 5.52 (1H, t), 6.8∼6.9 (2H, m), 7.00 (1H, t), 7.2∼7.4 (3H, m), 7.21 (1H, t), 8.00 (1H, d)

### [Example 1]

### 5,6,8,9,10,11-hexahydro-10-[(5-methyl-1H-imidazol-4-yl) methyl]-6-phenyl-11-oxo-4H-pyrido[3',4':4,5]pyrrolo[3,2,1-ij] quinoline:

A 650 mg (2.15 mmol) portion of the 5,6,8,9,10,11-hexahydro-6-phenyl-11-oxo-4H-pyrido[3',4':4,5]pyrrolo[3,2,1-ij] quinoline prepared in Reference Example 1 and 960 mg (2.58 mmol) of 4-(chloromethyl)-5-methyl-1-(triphenylmethyl)-1H-imidazole were dissolved in 20 ml of N,N-dimethylformamide, and then 120 mg (2.75 mmol) of 55% sodium hydride was added to the solution at room temperature. After stirring the mixture overnight at room temperature, it was poured into ice water and the resulting precipitate was collected by filtration. This precipitate was subjected to silica gel column chromatography and eluted with a mixed solvent of chloroform:ethanol = 400:3, and the eluate was then concentrated to obtain 424 mg (0.66 mmol) of 5,6,8,9,10,11-hexahydro-10-[(5-methyl-1-(triphenylmethyl)-1H-imidazol-4-yl)methyl]-6-phenyl-11-oxo-4H-pyrido[3',4':4,5]pyrrolo[3,2,1-ij]quinoline as a pale yellow glassy substance (31% yield).
¹H-NMR (δppm, CDCl₃, 270MHz):
1.47 (3H, s), 2.3∼2.6 (3H, m), 2.6∼2.9 (3H, m), 3.65 (2H, t), 4.57 (1H, d), 4.77 (1H, d), 5.48 (1H, t), 6.8∼6.9 (2H, m), 6.97 (1H, d), 7.1∼7.4 (20H, m), 8.01 (1H, d)

The 424 mg (0.66 mmol) of the above mentioned 5,6,8,9,10,11-hexahydro-10-[(5-methyl-1-(triphenylmethyl)-1H-imidazol-4-yl) methyl)-6-phenyl-11-oxo-4H-pyrido[3',4':4,5]pyrrolo[3,2,1-ij]quinoline was dissolved in a mixed solvent of 4 ml acetic acid, 4 ml tetrahydrofuran and 4 ml water, and the solution was heated under reflux for one hour and 30 minutes. After ice cooling the reaction solution, 1 N hydrochloric acid was added and then the mixture was washed with ether. After making the aqueous layer basic with an aqueous sodium hydroxide solution, the basic mixture was extracted with chloroform. The extract solution was dried and concentrated to obtain a residue, then the residue was subjected to silica gel column chromatography and eluted with a mixed solvent of chloroform:methanol = 50:1. The eluate was concentrated to obtain 137 mg (0.34 mmol) of the 5,6,8,9,10,11-hexahydro-10-[(5-methyl-1H-imidazol-4-yl)methyl]-6-phenyl-11-oxo-4H-pyrido[3',4':4,5]pyrrolo[3,2,1-ij]quinoline listed in the above heading, as a colorless powder (52% yield).
¹H-NMR (δppm, CDCl₃, 270MHz):
2.26 (3H, s), 2.3∼3.0 (6H, m), 3.57 (2H, m), 4.55 (1H, d), 4.56 (1H, d), 5.46 (1H, t), 6.7∼6.8 (2H, m), 7.00 (1H, d), 7.21 (1H, t), 7.2∼7.3 (3H, m), 7.45 (1H, s), 7.99 (1H, d)

### [Example 2]

### 5,6,8,9,10,11-hexahydro-10-[(5-methyl-1H-imidazol-4-yl) methyl]-6-phenyl-11-oxo-4H-pyrido[3',4':4,5]pyrrolo[3,2,1-ij] quinoline hydrochloride (compound 1):

The 137 mg of 5,6,8,9,10,11-hexahydro-10-[(5-methyl-1H-imidazol-4-yl)methyl]-6-phenyl-11-oxo-4H-pyrido[3',4':4,5]pyrrolo[3,2,1-ij]quinoline was dissolved in ethanol and hydrogen chloride gas was blown to this solution under ice cooling, after which the solution was concentrated to dryness under reduced pressure to obtain 120 mg of the 5,6,8,9,10,11-hexahydro-10-[(5-methyl-1H-imidazol-4-yl) methyl]-6-phenyl-11-oxo-4H-pyrido[3',4':4,5]pyrrolo[3,2,1-ij] quinoline hydrochloride listed in the above heading, as a powdery solid.
¹H-NMR (δppm, DMSO-d₆, 270MHz):
2.33 (3H, s), 2.3∼3.7 (8H, m), 4.54 (1H, d), 4.69 (1H, d), 5.79 (1H, t), 6.7∼6.9 (2H, m), 6.97 (1H, d), 7.12 (1H, t), 7.2∼7.4 (3H, m), 7.75 (1H, d), 8.90 (1H, s), 14.2 (2H, brs)

The pharmacological effects of the compound (compound 1) of the invention are demonstrated below. For comparison there are also provided the results of tests conducted by the same method on the following compounds:
(1) Ondansetron hydrochloride
   17) 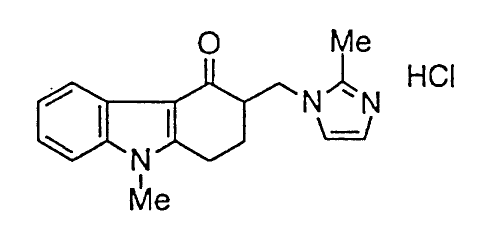
(2) YM-060
   18) 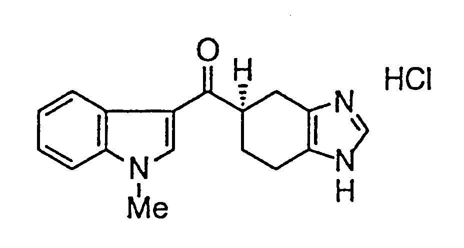
(3) Allosetron hydrochloride
   19) 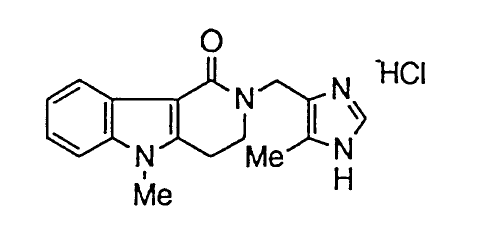
(4) 5,6,9,10-tetrahydro-10-[(5-methyl-1H-imidazol-4-yl)methyl]-4H-pyrido[3',4':4,5]pyrrolo[3,2,1-ij] quinolin-11(8H)-one (compound described in Japanese laid-open patent publication No. Hei-2-180885, hereunder referred to as "compound A")
   20)
(5) 4,5,7,8-tetrahydro-9-[(5-methyl-1H-imidazol-4-yl)methyl]-pyrido[4,3-b]pyrrolo[3,2,1-hi]indol-10(9H)-one (compound described in Japanese laid-open patent 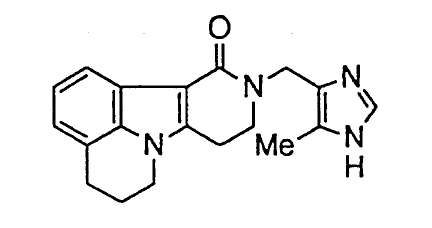 publication No. Hei-2-180885, hereunder referred to as "compound B")
   21) 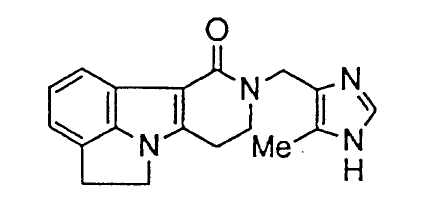
(6) 1,2,9,10-tetrahydro-8-[(5-methyl-1H-imidazol-4-yl)methyl]pyrido[3',4':4,5]pyrrolo[1,2,3-de][1,4]benzoxadin-7(8H)-one (compound described in Japanese laid-open patent publication No. Hei-2-180885, hereunder referred to as "compound C")
   22) 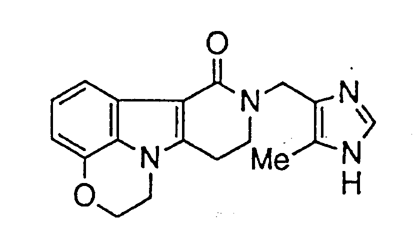
(7) 4,5,7,8,9,10-hexahydro-10-[(5-methyl-1H-imidazol-4-yl)methyl]-11H-azepino[4,3-b]pyrrolo[3,2,1-hi]indol-11-one (compound described in Japanese laid-open patent publication No. Hei-2-180885, hereunder referred to as "compound D")
   23) 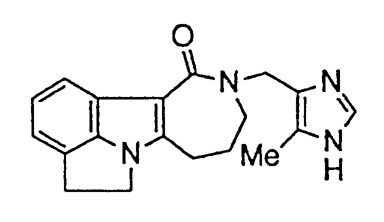
(8) 11-[(5-methyl-1H-imidazol-4-yl)methyl]-4,5,6,7,9,10,11,12-octahydro-pyrido[3',4':4,5]pyrrolo[3,2,1-jk][1]benzazepine (compound described in Japanese laid-open patent publication No. Hei-2-258785, hereunder referred to as "compound E")
   24) 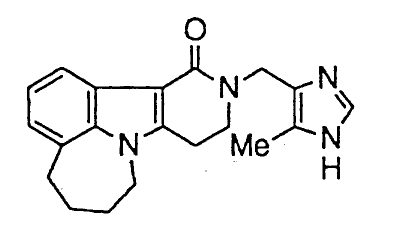
(9) 11-[(5-methyl-1H-imidazol-4-yl)methyl]-5,6,9,10,11,12-hexahydro-4H,8H- azepino[3',4':4,5]pyrrolo[3,2,1-ij]quinolin-12-one (compound described in Japanese laid-open patent publication No. Hei-2-258785, hereunder referred to as "compound F")
   25) 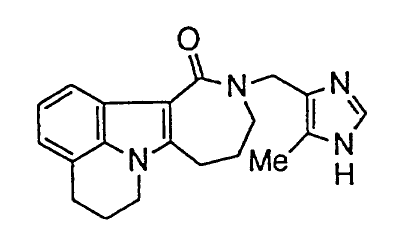
(10) 3-[(4-methyl-1-H-imidazol-5-yl)methyl]-1,2,3,4,8,9-hexahydro-pyrido[4',3':2,3]indolo[1,7a,7-ab][1]benzazepin-4-one (compound described in Japanese laid-open patent publication No. Hei-2-258785, hereunder referred to as "compound G")
   26) 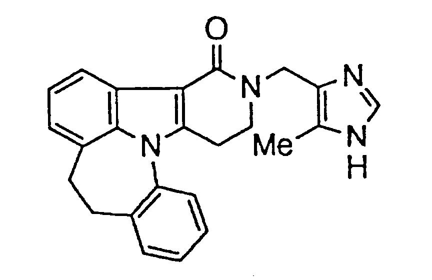
(11) 10-(endo-8-methyl-8-azabicyclo-[3,2,1]oct-3-yl-5,6,8,9,10,11-hexahydro-4H-pyrido[3',4':4,5]pyrrolo[3,2,1- ij]quinolin-11-one (compound described in Japanese laid-open patent publication No. Hei-2-258785, hereunder referred to as "compound H")
   27) 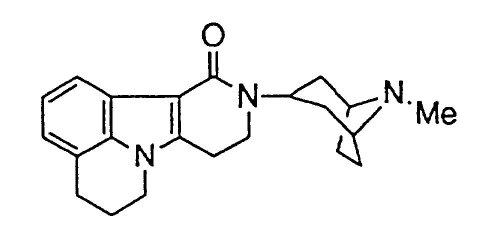
(12) 10-(1-azabicyclo-[2,2,2]oct-3-yl)-5,6,8,9,10,11-hexahydro-4H-pyrido[3',4':4,5]pyrrolo[3,2,1-ij]quinolin-11-one (compound described in Japanese laid-open patent publication No. Hei-2-258785, hereunder referred to as "compound I")
   28)

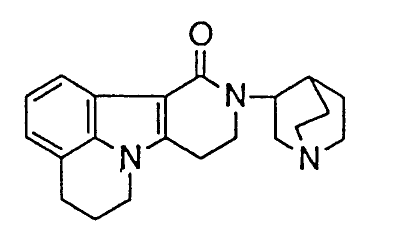

### [Test 1]

### Colonic 5-HT₃ receptor antagonism:

The test was conducted following the method of Miyata, et al. (The Journal of Pharmacology and Experimental Therapeutics Vol.259, No.1, 15 (1991)).

Male Hartley guinea pigs with a body weight of 400-500 g were bled to death, the distal colons (3-10 cm from the anus) were isolated and suspended in a Magnus tube filled with Krebs solution aerated with a mixed gas (95% O₂, 5% CO₂), and subjected to a tensile load of 1 g. After constant contraction induced by addition of 2-methylserotonin (10⁻⁴ M) was obtained, the test agent was added and the pA₂ value of the test agent was calculated based on its inhibiting action on the initial value. The value (pA₂ value) is defined as the negative logarithm (-logM) of the molar concentration (M) of the antagonist required to reduce the effect of 2-methylserotonin at a concentration of 2 times the 2-methylserotonin concentration (ED₅₀) necessary to induce half of the maximum contraction reaction, to an effect induced by an ED₅₀ of 2-methylserotonin.

**Table 1**

| Test compound | pA₂ | M (×10⁻⁹ Mole) |
|---|---|---|
| Compound - 1 | 9.4 | 0.41 |
| Ondansetron hydrochloride | 6.6 | 227 |
| YM-060 | 8.4 | 4.5 |
| Allosetron hydrochloride | 7.7 | 21 |
| Compound - A | 8.0 | 10 |
| Compound - B | <8.0 | 10 |
| Compound - C | <8.0 | >10 |
| Compound - D | <8.0 | >10 |
| Compound - E | <8.0 | >10 |
| Compound - F | <8.0 | >10 |
| Compound - G | <8.0 | >10 |
| Compound - H | <8.0 | >10 |
| Compound - I | <8.0 | >10 |

As is clear from Table 1, the compound of the invention exhibited stronger (at least 24-fold) antagonism against colic 5-HT₃ receptors, which effect was unpredictable based on the activity of known compounds with relatively similar structures (compounds A, B, C, D, E, F, G, H, I). It had about 500-fold, 10-fold and 50-fold higher activity compared to Ondansetron, YM-060 and Allosetron, respectively.

### [Test 2]

### Antagonism against von Bezold-Jarisch reflex (BJ reflex):

This test was conducted following the method of Fozard, at al. (Arch. Pharmacology, 326, 36-44, 1984). Male SD rats with a body weight of around 300 g were anesthetized by intraabdominal administration of 1.25 g/kg urethane, and an intravenous injection cannula was inserted in the right femoral vein while a sphygmomanometric cannula was inserted into the left femoral artery. The pulse was determined by measuring the electrocardiogram R waves (secondary induction) with an instantaneous pulse rate meter. After confirming satisfactory reproducibility of the reflexive bradycardia induced by intravenous administration of 30 µg/kg serotonin, the test compounds were intravenously administered 5 minutes prior to serotonin administration, the inhibition rates with respect to the initial values were found and the 50% inhibition doses (ID₅₀) were calculated.

Table 2 shows the results of Test 2 on compound 1. Also shown are the results for Ondansetron hydrochloride, YM-060, Allosetron hydrochloride and compound A which exhibited relatively high colonic 5-HT₃ receptor antagonism activity (pA₂ ≧ 8.0) in Test 1.

**Table 2**

| Test compound | ID₅₀ (µg/kg) |
|---|---|
| Compound-1 | 0.50 |
| Ondansetron hydrochloride | 2.0 |
| YM-060 | 0.026 |
| Allosetron hydrochloride | 0.28 |
| Compound-A | 0.11 |

As is clear from Table 2, the cardiac 5-HT₃ receptor antagonism was not particularly strong. Compound 1 exhibited activity on the heart receptors which was about 4 times that of Ondansetron, about 1/19 that of YM-060, about 1/1.8 that of Allosetron and about 1/4.5 that of compound A.

To summarize the results from Tests 1 and 2, the selectivity of compound 1 for colonic 5-HT₃ receptors was about 125 times that of Ondansetron (500 ÷ 4 = 125), about 190 times that of YM-060 (10 ÷ 1/19 = 190), about 90 times that of Allosetron (50 ÷ 1/1.8 = 90) and about 100 times that of compound A (24 ÷ 4.5 = 100).

Thus, the compound of the invention acted on colonic 5-HT₃ receptors at the lowest dose among the other 5-HT₃ receptor antagonists which were compared, and had a little effect on cardiac 5-HT₃ receptors at that dose.

### [Test 3]

### Inhibiting effect on restraint stress-induced diarrhea (oral administration):

This test was conducted following the method of Miyata, et al. (The Journal of Pharmacology and Experimental Therapeutics Vol.261, No.1, 297 (1992)). Nine to ten-week-old male Wistar rats starved overnight were placed in stress cages and their feces produced in 5 hours were observed. Of 15 rats, 14 developed diarrhea. The test compounds were administered orally one hour prior to placement in the stress cage, and the rate of inhibition on diarrhea was measured.

Table 3 shows the results of Test 3 on compound 1. Also shown are the results for YM-060 and compound A, which exhibited relatively high activity in Test 1.

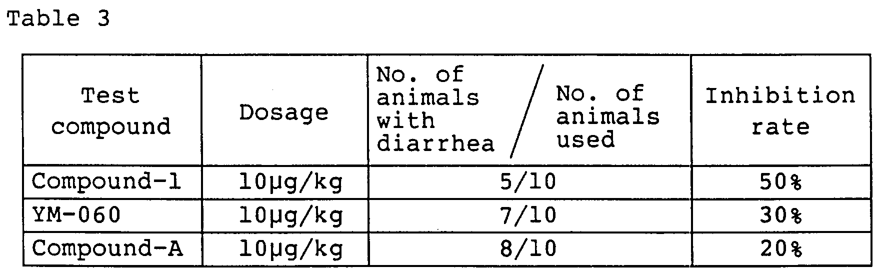

As is clear from Table 3, the compound of the invention exhibited a very powerful antidiarrheal effect.

### [Test 4]

### Duration of action:

The BJ reflex inhibition was measured in the same basic manner as in Test 2. However, serotonin was administered subsequent to intravenous administration of the test compounds (1 µg/kg), first at 5 minutes and then every 30 minutes thereafter, the BJ reflex was measured, and the inhibition rates with respect to the initial values were calculated and used as the indexes of duration of action.

Fig. 1 shows the results of Test 4 for compound 1. Also shown are the results for YM-060 which exhibited relatively high activity in Tests 1 and 2.

As is clear from Fig. 1, the compound of the invention was also highly superior in terms of duration of action.

### Industrial Applicability

Thus, the compounds of the present invention not only exhibit the same effect as known 5-HT₃ receptor antagonists, but also have more powerful and selective colonic 5-HT₃ receptor antagonism than known 5-HT₃ receptor antagonists, as well as a more powerful antidiarrheal effect and prolonged duration of action; they are hence useful as prophylactic or therapeutic agents against irritancy or vomiting induced by chemotherapy or radiation, reflux esophagitis, gastritis, migraine, combined headache, neuralgia, anxiety, psychosis, schizophrenia, memory disturbance, amnesia, dementia, rider's vertigo, arrhythmia, postoperative irritancy or vomiting, alcohol-, nicotine-or narcotic-dependency, skin itching, etc., and are particularly useful as prophylactic or therapeutic agents against irritable bowel syndrome, stress-related diarrhea, intestinal trauma-related diarrhea, radiation-induced diarrhea, antitumor agent-induced diarrhea, accelerated defecation, constipation, gastrointestinal motor disturbance and enteritis, as well as the abdominal pain accompanying them.

## Claims

1. An indole derivative represented by the general formula:
29) 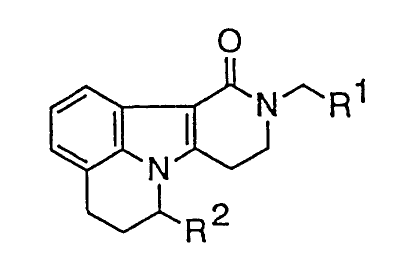 wherein R¹ represents:
30) 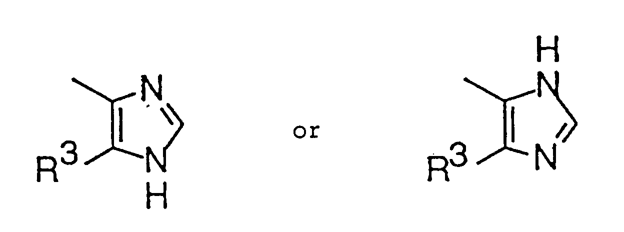 R² represents a phenyl group which may be substituted, and R³ represents a hydrogen atom or a methyl or ethyl group, either as a single isomer or as an isomer mixture, or a physiologically acceptable salt thereof, or its solvate.

2. An indole derivative according to claim 1 wherein R² is a phenyl group, either as a single isomer or as an isomer mixture, or a physiologically acceptable salt thereof, or its solvate.

3. An indole derivative according to claim 1 which is 5,6,8,9,10,11-hexahydro-10-[(5-methyl-1H-imidazol-4-yl) methyl]-6-phenyl-11-oxo-4H-pyrido[3',4':4,5]pyrrolo[3,2,1-ij] quinoline, either as a single isomer or as an isomer mixture, or a physiologically acceptable salt thereof, or its solvate.
